# EUROPÄISCHE PATENTSCHRIFT

(11) **EP 0 385 391 B1**
(45) Veröffentlichungstag und Bekanntmachung des Hinweises auf die Patenterteilung: **02.02.2000**
(21) Anmeldenummer: 90103790.3
(22) Anmeldetag: 27.02.1990
(51) Int. Cl.: C12N 15/81, C12N 15/54

(54) **Expressionsvektoren zur Synthese von Proteinen in der Spalthefe Schizosaccharomyces pombe**
Expression vectors for protein synthesis in Schizosaccharomyces pombe yeast
Vecteurs d'expression pour la synthèse de protéine chez la levure Schizosaccharomyces pombe

(30) Priorität: 02.03.1989 DE 3906540
(43) Veröffentlichungstag der Anmeldung: 05.09.1990
(73) Patentinhaber: HOECHST AKTIENGESELLSCHAFT, 65929 Frankfurt am Main (DE)
(72) Erfinder: Bröker, Michael, Dr., D-3550 Marburg (DE)

(56) Entgegenhaltungen:
- EP-A- 0 139 383
- EP-A- 0 268 772
- EP-A- 0 284 044
- NUCLEIC ACIDS RESEARCH Band 15, Nr. 18, 1987 Seiten 7369-7379, Arlington, Virginia, US; P. MERTINS et al.: "A single intronless actin gene in the fission yeast Schizosaccharomyces pombe: nucleotide sequence and transcripts formed in homologues and heterologues yeast"
- BIOTECHNIQUES Band 6, Nr. 8, 1988, Seite 734, Nattick, US; M. BROEKER: "Improved "ATG vector" series for bacterial synthesis of proteins and protein fragments"
- FEBS LETTERS Band 248, Nr. 1-2, 1989, Seiten 105-110, Amsterdam, NL; M. BOEKER et al.: "New expression vectors for the fission yeast Schizosaccharomyces pombe"

## Beschreibung

Die Erfindung betrifft besonders geeignete Expressionsvektoren zur Synthese von Proteinen in der Spalthefe Schizosaccharomyces pombe (S. pombe). Diese Expressionsvektoren sind neben anderen vorteilhaften Elementen mit einem starken homologen Promotor ausgerüstet.

Mit Hilfe gentechnologischer Methoden ist es gelungen, sowohl homologe als auch heterologe Proteine in großen Mengen in Hefen zu synthetisieren. Als Wirt fand bisher insbesondere die Bäckerhefe Saccharomyces cerevisiae Beachtung, weil für diese Hefe als erstes Vektorsysteme und Transformationmethoden ausgearbeitet worden sind. Für andere Hefen, wie z. B. Vertreter der Gattungen Kluyveromyces, Hansenula, Pichia und Schwannomyces wurden, da über diese Hefen weniger generelle Kenntnisse zur klassischen und Molekulargenetik vorlagen, erst in letzter Zeit Expressionssysteme erarbeitet. Zur Synthese von heterologen Proteinen in Spalthefe S. pombe liegen bisher nur sehr wenige Berichte vor. S. pombe ist eine der insgesamt vier Arten der Gattung Schizosaccharomyces (Lindner). Die anderen drei Arten sind: S. japonicus, S. malidovorans und S. octosporus. Nur von der Art S. pombe gibt es auxotrophe Stämme, die gentechnologische Arbeiten in Analogie zur Bäckerhefe erlauben.

S. pombe steht hinsichtlich der Evolution höher als die Bäckerhefe und ähnelt in mancher Hinsicht eher den Säugerzellen als der Bäckerhefe. So konnten z. B. Lee und Nurse (Nature 327, (1987) pp 31-35) das menschliche Gen, das Homologie zu dem cdc2 Gen von S. pombe hat, durch Komplementation in diesem Organismus identifizieren. Die Zellwände der Bäckerhefe bestehen vorwiegend aus Kohlenhydraten, insbesondere aus Mannan und Chitin. Spalthefen besitzen außer β-Glucan (46-54%) und alpha-Glucan (28%) im Gegensatz zu Bäckerhefen auch Galactomannan (9-14%) in der Zellwand, und zwar an der Peripherie der Zellwand und nahe dem Plasmalemma. Das Galactomannan besteht aus einem (1-6)-verknüpftem Rückgrat mit (1-2)-verknüpften Mannoseeinheiten und endständigen Galaktoseresten. Die Invertase von S. pombe ist ein Glykoprotein, das terminal Galaktoseeinheiten besitzt, während der Kohlenhydratanteil der Invertase aus Bäckerhefe nur aus Mannose aufgebaut ist. Die Fähigkeit der Spalthefen, komplexere Kohlenhydrate und Glykoproteine als die Bäckerhefe aufbauen zu können, kann ein entscheidendes Kriterium für die biologische Aktivität, der Stabilitat und der Antigenität von rekombinanten Proteinen sein. Ein weiterer Vorteil von S. pombe gegenüber der Bäckerhefe in biotechnischen Verfahren kann die Eigenschaft sein, daß sie noch bei 37°C-42°C zu wachsen vermag. Um einen bestimmten Organismus als Wirt für die gentechnische Herstellung von Proteinen nutzen zu können, müssen der Organismus als auch die Klonierungsvektoren bestimmte Voraussetzungen erfüllen.

Folgende Merkmale sind wünschenswert:
1. Der Hefestamm sollte eine oder mehrere auxotrophe Marker besitzen, vorzugsweise Defekte in den biosynthetischen Enzymen des Aminosäure- oder Purin-Pyrimidinstoffwechsels.
2. Der Vektor sollte
   a) zur autonomen Replikation sogenannte "origins of replication" (ori) oder "autonomously replicating sequences (ars) sowohl für die Hefe als auch für eine prokaryontische Zelle, vorzugsweise für E. coli besitzen
   b) über einen starken Promotor zur Initiation der gewünschten mRNA-Transkription verfügen
   c) einen Transkriptionsterminator besitzen, damit das exprimierte mRNA Transkript eine definierte Länge besitzt
   d) über singuläre Restriktionsschnittstellen zwischen dem Promotor und dem Terminatorelement zum Einklonieren der Fremd-DNA verfügen
   e) ein Resistenzgen zur Selektionierung in Bakterien besitzen (z. B. Ampicillinresistenz)
   f) Gene zur Komplementation der auxotrophen Marker des Stammes besitzen, die dem Stamm die entsprechende Prototrophie verleihen
   g) trotz all der gewünschten Funktionen nicht zu groß sein, weil sonst z. B. die Transformationseffizienz zu sehr sinkt.

Von den hier aufgeführten Kriterien werden die Wirt-spezifischen Eigenschaften durch drei S. pombe Stämme erfüllt, die Defekte im Uracil- bzw. im Leucin- oder Arginin-Stoffwechsel aufweisen. So ist ein S. pombe Stamm verfügbar, der defizient für die Orotidin 5′-Phosphatdecarboxylase ist (ura4). Ein anderer Stamm synthetisiert keine intakte Isopropylmalatdehydrogenase mehr (Leu1). Der ura4-Marker von S. pombe kann durch das entsprechende Ura3-Gen von S. cerevisiae komplementiert werden, und das Leu1-Gen von S. pombe durch das Leu2-Gen der Bäckerhefe. Ein Arg7-1 S. pombe Stamm ist durch das klonierte Arg7-Gen, das für die Argininosuccinatlyase kodiert, komplementierbar.

Transformationen von S. pombe mit einem Plasmid (pDB248) wurden erstmals durch Beach and Nurse (Nature 290, (1981) pp 140-142) beschrieben. Der von ihnen konstruierte Vektor besitzt ein Element, das das Plasmid zur autonomen Replikation befähigt, doch zeigte sich, daß dieser Vektor nicht stabil in den Zellen verbleibt und während der mitotischen Segregation verlorengeht. Ein anderer Vektor (pFL20), der von Losson und Lacroute (1983) Cell 32, 371-377, entwickelt worden ist, enthält zusätzlich zum ars-Element eine DNA-Einheit (stb), die bewirkt, daß das Plasmid in der Mitose symmetrisch an Mutter- und Tochterzelle weitergegeben wird. Während der Vektor pDB248 leu1-Stämmen eine Leucin-Prototrophie vermittelt, verleiht das Plasmid pFL20 ura4-Stämmen die Fähigkeit zur Synthese von Uracil. Die jeweiligen mRNA-Transkripte werden durch Promotoren der Bäckerhefegene in der Spalthefe initiiert. Diese Beispiele zeigen, daß-Transkriptionsstartsignale der Bäckerhefe in der Spalthefe funktionell sind.

Die Transkriptionsstarts von Bäckerhefe-Genen sind jedoch nicht identisch mit den entsprechenden Stellen im homologen System bei S. pombe (Russel, (1983) Nature 301, pp 167-169). Ebenso existieren Unterschiede bei der Transkriptionstermination.

In EP-A-0 139 383 wird ein Vektor zur Expression von Fremdgenen in S. pombe beschrieben, der allerdings nur unter Selektionsdruck mitoxisch stabil ist.

Wir haben gefunden, daß die Expression von Fremdgenen in S. pombe gesteigert werden kann, wenn Promotoren und Terminatoren aus S. pombe verwendet werden. Dies gilt ganz allgemein für die Verwendung der homologen Promotoren und Terminatoren bei Schizosaccharomyces.

Die Erfindung betrifft folglich Vektoren zur Expression von Fremdgenen in S. pombe, wobei diese Vektoren einen S. pombe Promotor, vorzugsweise den Promotor des S. pombe Alkoholdehydrogenase-Gens, einen S. pombe Terminator, vorzugsweise den des S. pombe Actin-Gens und einen Replikationsorigin und das "stb"-Element für S. pombe neben gegebenenfalls anderen Elementen eines Vektorgerüsts enthalten. Diese anderen Elemente sind einzeln oder in Kombination ein Antibiotika-Resistenz-Gen in E. coli, einen Replikationsorigin in E. coli und eine komplementierendes Gen für die ausgewählte Auxotrophie des S. pombe-Wirts, vorzugsweise das Ura3-Gen von S. cerevisiae zur Komplementation des Ura4-Defektes von S. pombe, aber auch z. B. das Arg7-Gen von S. pombe oder das Leu2-Gen von S. cerevisiae.

Die Erfindung ist ferner in den Beispielen und den Patentansprüchen offenbart.

### Beispiele:

### 1. Vektorkonstruktion

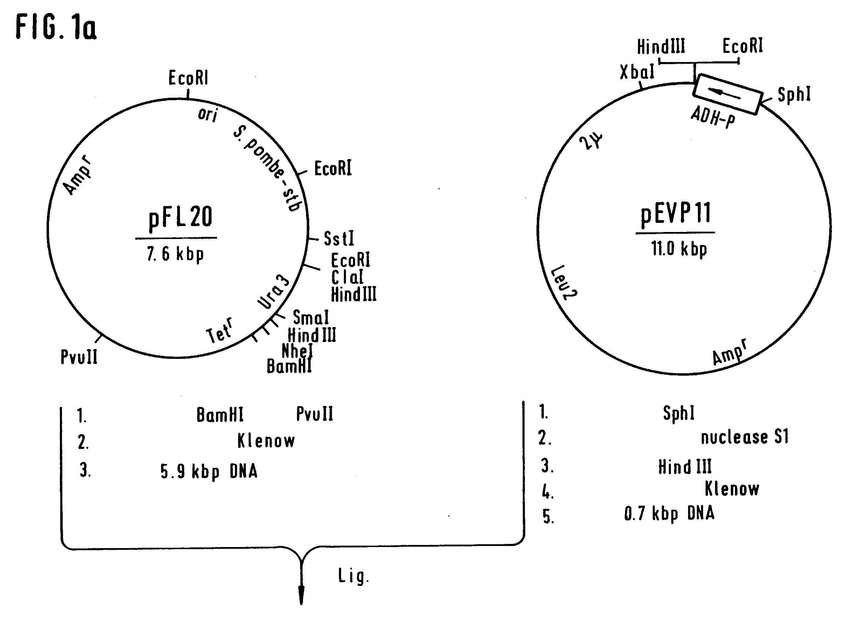

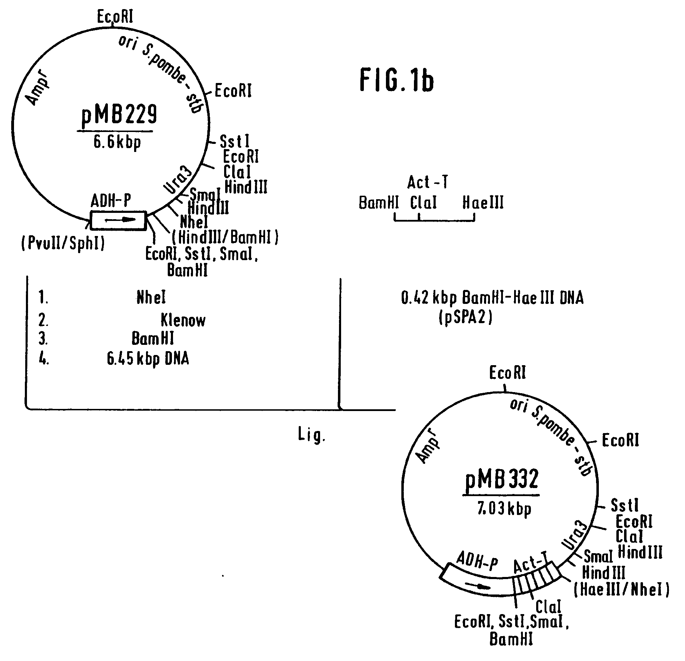

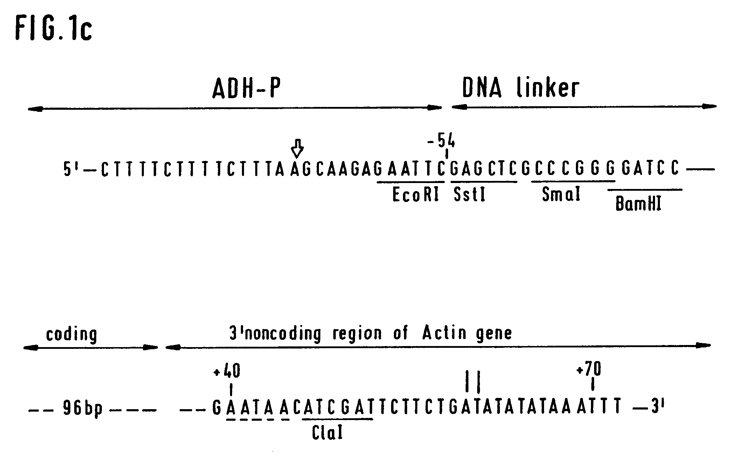

Der Vektor pFL20 diente als Grundlage für die Vermittlung der Ampicillin-Resistenz in E. coli, der "ori"-Funktion in E. coli, der "ori"- und der "stb"-Funktion in S. pombe und des Ura3-Gens von S. cerevisiae zur Komplementation des ura4-Gens von S. pombe. Das Plasmid pFL20 wurde mit BamHI und PvuII verdaut, die überhängenden 5'-Enden mit Polymerase (Klenow Fragment) aufgefüllt und das ca. 5,9 kbp große DNA-Fragment isoliert. In diesen präparierten Vektor, der nunmehr nicht mehr das ursprüngliche Tetrazyklinresistenzgen trägt, wurde die Kontrollregion (Promotor) des Alkoholdehydrogenase (ADH)-Gens von S. pombe einkloniert, innerhalb der die Erkennungsstellen für die Initiation der Transkription liegen. Hierzu wurde ein 0,7 kbp großes DNA-Fragment verwendet, das 5'-endig vor dem Translationsstartsignal des ADH-Gens liegt und mit der EcoRI-Stelle bis zur Position -54 bp vor dem ATG Startcodon endet. Als Quelle für das ADH-Promoterfragment diente das 0,7 kbp große SphI-HindIII DNA Fragment aus dem Plasmid pEVP11, welches seinerseits dadurch entstanden war, daß das EcoRI-SphI DNA Fragment, das den S. pombe ADH-Promotor trägt, zusammen mit dem EcoRI-HindIII Polylinker Fragment von pUC12 in den S. cerevisiae-E.coli shuttle Vektor YEp13 nach Verdauung mit EcoRI und SphI einkloniert worden war (Fig. 1).

Der neu entstandene Vektor pMB229 hat gegenüber dem Plasmid pEVP11 den Vorteil, daß 1. er sich in S. pombe stabil autonom replizieren kann, 2. nichtessentielle DNA-Bereiche, wie kodierende Bereiche des E. coli Tetrazyklingens und der S. cerevisiae 2 _ DNA entfernt worden sind und 3. der Vektor mit ca. 6,6 kbp als Hefevektor recht klein ist. Die singuläre BamHI-Stelle 3'-endig vom ADH-Promotor bietet die Möglichkeit, fremde Gene einzuklonieren und damit die Expression des Fremdgenes unter die Kontrolle des ADH-Promotors zu stellen.

3'-endig vom ADH-Promotor wurde eine Transkriptionsterminationseinheit in den Vektor pMB229 eingefügt. Dazu wurde das 0.42 kbp große BamHI-HaeIII DNA-Fragment des S. pombe Actin-Genes, das den Transkriptionsterminator trägt, aus dem Plasmid pSPA2 (Mertins und Gallwitz, Nucleic Acids Research 15, 7369-7379 (1987)) isoliert und in pMB229 einkloniert. Dazu wurde pMB229 zuerst mit NheI verdaut, die überstehenden 5'-Enden mit Polymerase I (Klenow) aufgefüllt, das Plasmid mit BamHI nachverdaut, und das 6,45 kbp große DNA-Fragment isoliert und mit dem 0,42 kbp großen DNA-Fragment aus pSPA2 ligiert. Damit besitzt das neue Plasmid pMB332 (Fig. 1) eine Transkriptionsinitiations- und Terminationseinheit aus zwei verschiedenen S. pombe Genen, nämlich aus dem ADH-Gen und aus dem Actin-Gen. Die Transkription im ADH-Promotoranteil sollte 24 bp vor der singulären BamHI-Stelle beginnen, so daß bei Einklonierung eines Fremdgenes eine Hybrid mRNA gebildet wird, die Anteile trägt aus dem ADH-Gen, dem DNA-Linker, der ursprünglich aus pUC12 stammt, und der DNA, die in den Vektor pMB332 einkloniert wird. Zwischen dem Transkriptionsstart und der BamHI-Stelle liegt kein Translationsstartcodon, so daß das erste ATG-Triplett der rekombinanten mRNA als Translationsstart fungieren kann. Die Signale für die Terminierung der Transkription liegen 8 bp 3'-endig von der ClaI-Stelle. Im Actin-Gen wird bei den bp +59 und +60 terminiert. Im Vektor pMB332 liegen diese Terminationsstops 117 und 118 bp 3'-endig von der singulären BamHI-Stelle, so daß abhängig von der einklonierten Fremd-DNA ein entsprechend langer 3'-nichtranslatierter Bereich auf der rekombinanten mRNA vorliegt, es sei denn, auf der einklonierten Fremd-DNA liegen effiziente Transkriptionsstopsignale.

### Beispiele 2 und 3:

Die Anwendbarkeit des Expressionsvektors zur Synthese fremder Proteine in S. pombe wurde an einem prokaryotischem Protein, der beta-Galaktosidase (betagal) von E. coli, und einem eukaryotischen Protein, dem menschlichen Gerinnungsfaktor F XIIIa, gezeigt.

### Beispiel 2: Synthese von Beta-Galaktosidase

Zur Synthese von betagal in S. pombe wurde das Plasmid pMB334 hergestellte Die Konstruktion erfolgte folgendermaßen: Das betagal kodierende ca. 3000 bp große HindIII-NcoI DNA Fragment des Vektors pLG400 (Guarente et al., Cell 20, (1980) 543-553) wurde mit Polymerase I Klenow-Fragment behandelt und in die singuläre, ebenfalls PolI behandelte BamHI-Stelle von pMB332 kloniert. Bei dem neuen Plasmid pMB334 steht die Transkription der betagal kodierende DNA nun unter der Kontrolle des ADH Promotors. Der Vektor pMB334 wurde in den Uracil auxotrophen Stamm S. pombe ura4-294 nach der Methode von Bröker (Biotechniques 5, (1987) 516-518) transformiert. In Zellextrakten dieser Transformanten konnte im Testansatz nach Miller enzymatisch aktive betagal nachgewiesen werden und S. pombe (pMB334)-Kolonien verfärben sich auf Agar-Nährmedien mit dem betagal Indikator X-gal blau, während nichttransformierte Zellen farblos bleiben.

### Beispiel 3: Synthese von Faktor (F) XIIIa

Zur Synthese von FXIIIa in S. pombe wurde das Plasmid pMB333 hergestellt. Die Konstruktion wurde folgendermaßen vorgenommen: Das ca. 2400 bp große EcoRI-HindIII DNA Fragment des Vektors pTrc99A-FXIIIa (Amann et al., Gene 69, 301-315, 1988), das die cDNA für FXIIIa trägt, wurde mit PolI behandelt und in die singuläre, ebenfalls PolI behandelte BamHI-Stelle des Plasmid pMB332 ligiert. In dem neuen Plasmid steht die Transkription der FXIIIa cDNA unter der Kontrolle des ADH Promotors. Der Vektor pMB333 wurde in S. pombe ura4-294 transformiert. In Extrakten von Zellen, die in einem komplexen Medium in Schüttelkulturen angewachsen waren, konnten in einem spezifischen ELISA 2 mg FXIIIa/l nachgewiesen werden. Im Aktivitätstest nach Karges (in: Methods in Enzymatic Analysis, Vol. 5, pp 400 - 405, (Bergmeyer, H. U. ed.), 1984) wurde die gleiche Konzentration an FXIIIa ermittelt. In Western Blot-Analysen konnte gezeigt werden, daß das FXIIIa aus Hefen das gleiche Laufverhalten wie plazenteres FXIIIa besitzt und somit vom Molekulargewicht identisch ist.

### Beispiel 4: Konstruktion eines S. pombe "ATG-vektors"

Wenn Proteinfragmente exprimiert werden sollen, die aminoterminal kein Methionin besitzen, das durch das Translationsstartcodon ATG kodiert ist, kann dieses Signal durch den Vektor vorgegeben werden. Dazu wurde das Plasmid pMB332 zu einem "ATG-Vektor" (pMB340) umgewandelt.

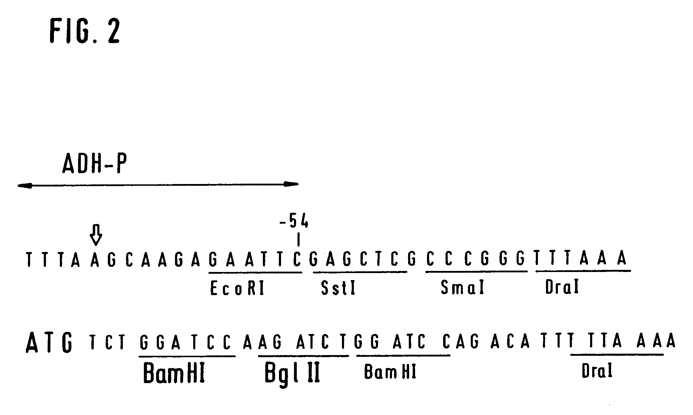

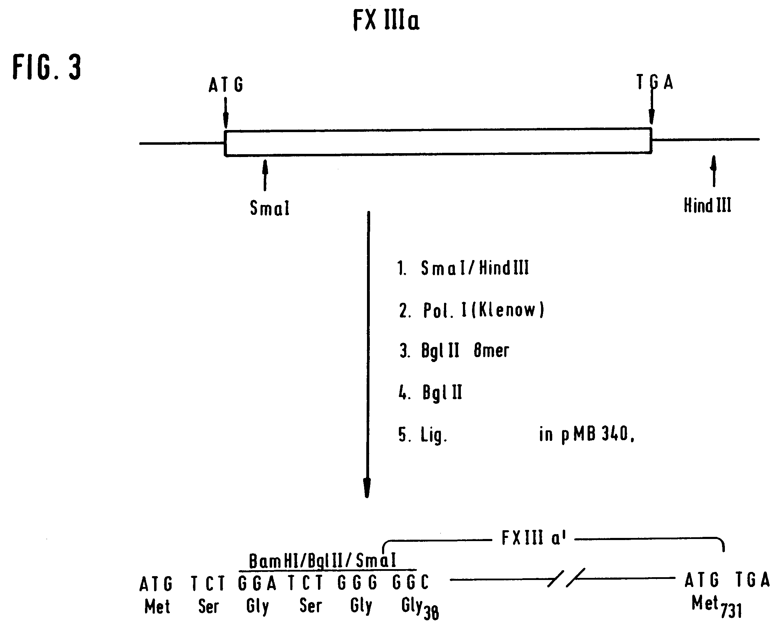

Im folgenden wurde pMB332 mit BamHI geöffnet und die überstehenden DNA-Einzelstränge wurden mit Sojabohnen S1-Nuklease abverdaut. Daraufhin wurde ein Oligonukleotid eingebaut, das ein ATG-Translationsstart-Signal beinhaltet (siehe Fig. 2). Dem ATG-Codon folgen eine BamHI und eine BglII-Stelle, in die Fremd-DNA eingefügt werden kann, dergestalt, daß die Protein-kodierende Sequenz im Leserahmen mit dem ATG_{Met}-Codon ist. Direkt nach dem ATG-Triplett folgt ein TCT-Codon, das für Serin kodiert. Dadurch soll gewährleistet sein, daß das exprimierte Protein eine lange Halbwertszeit besitzt; denn viele Proteine, die aminoterminal mit Met-Ser beginnen, besitzen eine lange Halbwertszeit. Von derartigen Proteinen wird das Methionin durch die Methioninaminopeptidase abgespalten und das Serin durch einen N-Acetyltransferase modifiziert. Ein primäres Translationsprodukt, das durch den Vektor pMB340 kodiert wird, hat demnach die Formel Met-Ser-Polypeptid, wobei das rekombinante Protein posttranslational in Ac-Ser-Polypeptid modifiziert werden kann.

Im weiteren kann in den Vektor pMB340 einklonierte DNA mit DraI herausgeschnitten werden und in andere Vektoren als Kassette eingefügt werden. Bei dem Transfer wird auch die Basenpaarfolge AAA, die sich 5' vor dem ATG-Translationsstart-Codon befindet, überführt, was den Vorteil bietet, daß in Position -3 vor dem Translationsstart auf der mRNA ein A vorhanden ist, was eine effiziente Translation bewirkt. Der Vektor pMB340 bietet also die Möglichkeit, jedes Proteinfragment effizient in S. pombe zu exprimieren, wobei je zwei bis sechs Aminosäuren zusätzlich am Aminoterminus des rekombinanten Proteins befindlich sind.

### Beispiel 5: Expression von FXIIIa' mit Hilfe des "ATG-Vektors" pMB340

FXIIIa wird durch Thrombin zu enzymatisch aktivem FXIIIa' umgewandelt. Hierbei wird die aminoterminale Peptidsequenz (Aktivierungspeptid) zwischen dem Arg₃₇ und dem Gly₃₈ gespalten. Innerhalb dieser Thrombin-Spaltstellen befindet sich auf der Ebene der cDNA eine singuläre SmaI-Schnittstelle, mit der man den kodierenden cDNA-Bereich des FXIIIa' vom kodierenden Bereich des Aktivierungspeptids abspalten kann. Im folgenden wurde die FXIIIa cDNA mit SmaI und HindIII verdaut, die HindIII-Stelle durch Polymerase I Klenow in Anwesenheit von dNTPs aufgefüllt und an die nunmehr für FXIIIa' kodierende cDNA ein BglII 8mer Linker (5'-CAGATCTG-3') anligiert. Nach Verdau mit BglII wurde die FXIIIa'-spezifische cDNA in mit BamHI linearisiertes pMB340 ligiert. Der neue Vektor pMB357 kodiert somit für ein bereits aktiviertes FXIIIa, das, vorgegeben durch den Vektor und die Klonierungsprozedur, fünf zusätzliche Aminosäuren am Aminoterminus besitzt.

Dieses in S. pombe synthetisierte modifizierte FXIIIa' hatte bereits ohne Aktivierung von Thrombin Transglutaminase-Aktivität.

Diese Beispiele belegen, daß man mit Hilfe der neu geschaffenen Expressionsplasmide pMB332 und pMB340 fremde prokaryotische und eukaryontische Proteine in aktiver Form in S. pombe herstellen kann. Im weiteren kann man zusätzliche Restriktionsstellen 3'-endig vom ADH-Promotor einfügen, um z.B. Ligierungen von Fremd-DNA in gerichteter Orientierung vornehmen zu können. Durch Einfügen einer DNA-Sequenz mit der Basenfolge ATG kann man ein Signal für die Inititation der Translation vorgeben und somit auch Genfragmente ohne ein eigenes Translationsstartsignal zur Expression bringen. Da die Halbwertszeit des fremden Proteins und posttranslationale Modifikationen Stamm-abhängig sein können, kann man durch Austausch des ura-Markers im Plasmid pMB332 gegen z.B. den Leu2-Marker oder Arg7-Marker den Expressionsvektor dahingehend modifizieren, daß man auch Leu2 oder Arg7 auxotrophe Stämme von S. pombe transformieren kann.

## Patentansprüche (Patentansprüche für folgende(n) Vertragsstaat(en): AT, BE, CH, DE, DK, FR, GB, IT, LI, LU, NL, SE)

1. Expressionsvektoren zur Expression von Fremdgenen in der Gattung Schizosaccharomyces (S.), die neben einem Vektorgerüst, enthaltend das "stb"-Element, als weitere Elemente
(a) einen Promotor der Gattung Schizosaccharomyces und
(b) einen Terminator der Gattung Schizosaccharomyces enthalten.

2. Expressionsvektoren nach Anspruch 1, wobei (a) und (b) jeweils zur Wirtsart homolog sind.

3. Expressionsvektoren nach Anspruch 2 zur Expression von Fremdgenen in S. pombe, wobei
(a) ein S. pombe-Promotor und
(b) ein S. pombe-Terminator ist.

4. Expressionsvektoren nach Anspruch 3, wobei (a) der S. pombe ADH-Promotor und (b) der Terminator des S. pombe Actin-Gens ist.

5. Expressionsvektor pMB332 nach Anspruch 1 gemäß Fig. 1, dadurch gekennzeichnet, daß er eine Replikationssequenz für E. coli, einen Selektionsmarker für E. coli, eine Replikationssequenz für S. pombe, ein "stb"-Element von S. pombe, einen S. pombe-Promotor, einen S. pombe-Terminator und das URA3-Gen von S. pombe sowie eine singuläre BamH1-Schnittstelle zwischen dem Promotor und dem Terminator enthält.

6. "ATG"-Expressionsvektor nach Anspruch 1, dadurch gekennzeichnet, daß er eine Replikationssequenz für E. coli, einen Selektionsmarker für E. coli, eine Replikationssequenz für S. pombe, ein "stb"-Element von S. pombe, einen S. pombe-Promotor, einen S. pombe-Terminator und das URA3-Gen von S. pombe sowie eine singuläre Bgl II-Schnittstelle zwischen dem Promotor und dem Terminatur enthält und daß der Bgl II-Schnittstelle 10 bp flußaufwärts ein ATG-Translationsstartcodon vorangesetzt ist.

7. Verfahren zur gentechnischen Herstellung von Fremdproteinen in Vertretern der Gattung Schizosaccharomyces, dadurch gekennzeichnet, daß Vektoren nach Anspruch 1, 2, 3, 4, 5 oder 6 eingesetzt werden.

8. Verwendung eines Verfahrens nach Anspruch 7 zur Herstellung von Faktor XIIIa.

9. Verwendung eines Verfahrens nach Anspruch 7 zur Herstellung von Faktor XIIIa'.

## Patentansprüche (Patentansprüche für folgende(n) Vertragsstaat(en): ES)

1. Verfahren zur gentechnischen Herstellung von Fremdproteinen in Vertretern der Gattung Schizosaccharomyces, dadurch gekennzeichnet, daß Vektoren, die neben einem Vektorgerüst, enthaltend das "stb"-Element, als weitere Elemente
(a) einen Promotor der Gattung Schizosaccharomyces und
(b) einen Terminator der Gattung Schizosaccharomyces enthalten,
zum Einsatz kommen

2. Verfahren nach Anspruch 1, dadurch gekennzeichnet, daß (a) der S. pombe ADH-Promotor und (b) der Terminator des S. pombe Actin-Gens ist.

3. Verfahren nach Anspruch 1, dadurch gekennzeichnet, daß der Vektor eine Replikationssequenz für E. coli, einen Selektionsmarker für E. coli, eine Replikationssequenz für S. pombe, ein "stb"-Element von S. pombe, einen S. pombe-Promotor, einen S. pombe-Terminator und das URA3-Gen von S. pombe sowie eine singuläre BamH1-Schnittstelle zwischen dem Promotor und dem Terminator enthält.

4. Verfahren nach Anspruch 1, dadurch gekennzeichnet, daß der Vektor eine Replikationssequenz für E. coli, einen Selektionsmarker für E. coli, eine Replikationssequenz für S. pombe, ein "stb"-Element von S. pombe, einen S. pombe-Promotor, einen S. pombe-Terminator und das URA3-Gen von S. pombe sowie eine singuläre Bgl II-Schnittstelle zwischen dem Promotor und dem Terminator enthält und daß der Bgl II-Schnittstelle 10 bp flußaufwärts ein ATG-Translationsstartcodon vorangesetzt ist.

5. Verwendung eines Verfahrens nach Anspruch 1 zur Herstellung von Faktor XIIIa.

6. Verwendung eines Verfahrens nach Anspruch 1 zur Herstellung von Faktor XIII a'.

## Claims (Claims for the following Contracting State(s): AT, BE, CH, DE, DK, FR, GB, IT, LI, LU, NL, SE)

1. An expression vector for expressing foreign genes in the genus Schizosaccharomyces (S.), which comprises, in addition to a vector skeleton comprising the "stb" element,
(a) a promoter from the genus Schizosaccharomyces and
(b) a terminator from the genus Schizosaccharomyces,
as further elements.

2. An expression vector as claimed in claim 1, where (a) and (b) are in each case homologous with the host species.

3. An expression vector as claimed in claim 2 for expressing foreign genes in S. pombe, where (a) is an S. pombe promoter and (b) an S. pombe terminator.

4. An expression vector as claimed in claim 3, where (a) is the S. pombe ADH promoter and (b) is the terminator of the S. pombe actin gene.

5. An expression vector pMB332 as claimed in claim 1 in accordance with Figure 1, which comprises a replication sequence for E. coli, a selection marker for E. coli, a replication sequence for S. pombe, an "stb" element of S. pombe, an S. pombe promoter, an S. pombe terminator and the S. pombe URA3 gene, and a single BamHI cleavage site between the promoter and the terminator.

6. An "ATG" expression vector as claimed in claim 1, which comprises a replication sequence for E. coli, a selection marker for E. coli, a replication sequence for S. pombe, an S. pombe "stb" element, an S. pombe promoter, an S. pombe terminator an the S. pombe URA3 gene, and a single BglII cleavage site between the promoter and the terminator and in which an ATG translation start codon precedes the BglII cleavage site 10 bp upstream.

7. A method for the recombinant production of foreign proteins in the representatives of the genus Schizosaccharomyces, wherein vectors as claimed in claim 1, 2, 3, 4, 5 or 6 are employed.

8. The use of a method as claimed in claim 7 for the preparation of factor XIIIa.

9. The use of a method as claimed in claim 7 for the preparation of factor XIIIa'.

## Claims (Claims for the following Contracting State(s): ES)

1. A method for the recombinant production of foreign proteins in representatives of the genus Schizosaccharomyces, wherein vectors are employed which comprise, besides a vector skeleton comprising the "stb" element,
(a) a promoter from the genus Schizosaccharomyces and
(b) a terminator from the genus Schizosaccharomyces
as further elements.

2. A method as claimed in claim 1, wherein (a) is the S. pombe ADH promoter and (b) is the terminator of the S. pombe actin gene.

3. A method as claimed in claim 1, wherein the vector comprises a replication sequence for E. coli, a selection marker for E. coli, a replication sequence for S. pombe, an "stb" element of S. pombe, an S. pombe promoter, an S. pombe terminator and the S. pombe URA3 gene, and a single BamHI cleavage site between the promoter and the terminator.

4. A method as claimed in claim 1, wherein the vector comprises a replication sequence for E. coli, a selection marker for E. coli, a replication sequence for S. pombe, an S. pombe "stb" element, an S. pombe promoter, an S. pombe terminator and the S. pombe URA3 gene, and a single BglII cleavage site between the promoter and the terminator and in which an ATG translation start codon precedes the BglII cleavage site 10 bp upstream.

5. The use of a method as claimed in claim 1 for the preparation of factor XIIIa.

6. The use of a method as claimed in claim 1 for the preparation of factor XIIIa'.

## Revendications (Revendications pour l'(les) Etat(s) contractant(s) suivant(s): AT, BE, CH, DE, DK, FR, GB, IT, LI, LU, NL, SE)

1. Vecteurs d'expression pour l'expression de gènes étrangers dans le genre *Schizosaccharomyces* (*S*.), qui, outre un squelette de vecteur, contenant l'élément "stb", contiennent en tant qu'autres éléments
(a) un promoteur du genre *Schizosaccharomyces* et
(b) un terminateur du genre *Schizosaccharomyces.*

2. Vecteurs d'expression selon la revendication 1, dans lesquels (a) et (b) sont homologues chacun de l'espèce hôte.

3. Vecteurs d'expression selon la revendication 2, pour l'expression de gènes étrangers dans *S*. *pombe*, dans lesquels
(a) est un promoteur de *S*. *pombe* et
(b) est un terminateur de *S*. *pombe*.

4. Vecteurs d'expression selon la revendication 3, dans lesquels (a) est le promoteur ADH de *S*. *pombe* et (b) est le terminateur du gène d'actine de *S*. *pombe*.

5. Vecteur d'expression pMB332 selon la revendication 1, conforme à la figure 1, caractérisé en ce qu'il contient une séquence de réplication pour *E*. *coli*, un marqueur de sélection pour *E*. *coli*, une séquence de réplication pour *S*. *pombe*, un élément "stb" de *S*. *pombe*, un promoteur de *S*. *pombe*, un terminateur de *S*. *pombe* et le gène URA-3 de *S*. *pombe* ainsi qu'un unique site de coupure *Bam*HI entre le promoteur et le terminateur.

6. Vecteur d'expression "ATG" selon la revendication 1, caractérisé en ce qu'il contient une séquence de réplication pour *E*. *coli*, un marqueur de sélection pour *E*. *coli*, une séquence de réplication pour *S*. *pombe*, un élément "stb" de *S*. *pombe*, un promoteur de *S*. *pombe*, un terminateur de *S*. *pombe* et le gène URA-3 de *S*. *pombe* ainsi qu'un unique site de coupure *Bgl*II entre le promoteur et le terminateur, et en ce qu'un codon d'initiation de traduction ATG est disposé 10 pb en amont du site de coupure *Bgl*II.

7. Procédé pour la production par génie génétique de protéines étrangères dans des représentants du genre *Schizosaccharomyces*, caractérisé en ce que l'on utilise des vecteurs selon la revendication 1, 2, 3, 4, 5 ou 6.

8. Utilisation d'un procédé selon la revendication 7, pour la production du facteur XIIIa.

9. Utilisation d'un procédé selon la revendication 7, pour la production du facteur XIIIa'.

## Revendications (Revendications pour l'(les) Etat(s) contractant(s) suivant(s): ES)

1. Procédé pour la production par génie génétique de protéines étrangères dans des représentants du genre *Schizosaccharomyces*, caractérisé en ce que l'on utilise des vecteurs qui, outre un squelette de vecteur, contenant l'élément "stb", contiennent en tant qu'autres éléments
(a) un promoteur du genre *Schizosaccharomyces* et
(b) un terminateur du genre *Schizosaccharomyces*.

2. Procédé selon la revendication 1, caractérisé en ce que (a) est le promoteur ADH de *S*. *pombe* et (b) est le terminateur du gène d'actine de *S*. *pombe*.

3. Procédé selon la revendication 1, caractérisé en ce que le vecteur contient une séquence de réplication pour *E*. *coli*, un marqueur de sélection pour *E*. *coli*, une séquence de réplication pour *S*. *pombe*, un élément "stb" de *S*. *pombe*, un promoteur de *S*. *pombe*, un terminateur de *S*. *pombe* et le gène URA-3 de *S*. *pombe* ainsi qu'un unique site de coupure *Bam*HI entre le promoteur et le terminateur.

4. Procédé selon la revendication 1, caractérisé en ce que le vecteur contient une séquence de réplication pour *E*. *coli*, un marqueur de sélection pour *E*. *coli*, une séquence de réplication pour *S*. *pombe*, un élément "stb" de *S*. *pombe*, un promoteur de *S*. *pombe*, un terminateur de *S*. *pombe* et le gène URA-3 de *S*. *pombe* ainsi qu'un unique site de coupure *Bgl*II entre le promoteur et le terminateur, et en ce qu'un codon d'initiation de traduction ATG est disposé 10 pb en amont du site de coupure *Bgl*II.

5. Utilisation d'un procédé selon la revendication 1, pour la production du facteur XIIIa.

6. Utilisation d'un procédé selon la revendication 1, pour la production du facteur XIIIa'.
